(19) Europäisches Patentamt
European Patent Office
Office européen des brevets

(11) **EP 3 684 297 B1**

(12) **EUROPEAN PATENT SPECIFICATION**

(45) Date of publication and mention
of the grant of the patent:
**28.07.2021 Bulletin 2021/30**

(21) Application number: **18768920.3**

(22) Date of filing: **19.09.2018**

(51) Int Cl.:
*A61F 2/28* (2006.01)          *A61L 27/56* (2006.01)

(86) International application number:
**PCT/EP2018/075357**

(87) International publication number:
**WO 2019/057775 (28.03.2019 Gazette 2019/13)**

(54) **MICROARCHITECTURE OF OSTEOCONDUCTIVE BONE SUBSTITUTE**

MIKROARCHITEKTUR EINES OSTEOKONDUKTIVEN KNOCHENERSATZES

MICROARCHITECTURE DE SUBSTITUTION OSSEUSE OSTÉOCONDUCTIVE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **20.09.2017 EP 17192143
08.06.2018 EP 18176736**

(43) Date of publication of application:
**29.07.2020 Bulletin 2020/31**

(73) Proprietor: **Universität Zürich
8006 Zürich (CH)**

(72) Inventor: **WEBER, Franz
78224 Singen (DE)**

(74) Representative: **Schulz Junghans
Patentanwälte PartGmbB
Großbeerenstraße 71
10963 Berlin (DE)**

(56) References cited:
**EP-A2- 2 564 813          US-A1- 2011 307 073
US-A1- 2013 195 955**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

The present invention relates to a bone substitute material comprising a network of concave pores connected by channels.

Description

Bones have the natural ability to heal based on the presence of osteoinductive proteins in the bone matrix and mesenchymal progenitor and stem cell in the bone marrow, the periost and other surrounding tissues. If the defects surpass a critical size, a surgical intervention is often required to regenerate the bone by transplantation of autologous bone. Bone is the second most frequently transplanted tissue in Europe after blood, with about 1 million procedures annually. With the transplantation of bone, osteoinductive proteins and mesenchymal stem cells are transferred to the diseased site and support bone regeneration. The third driving force of bone repair is osteoconduction.

Osteoconduction defines a three-dimensional process observed when porous structures are implanted in or adjacent to bone. In a first phase, capillaries, perivascular tissues, and osteoprogenitor cells migrate into porous spaces and, secondly, incorporate the porous structure with newly formed bone. In 1893, osteoconduction was described based on histologies from autologous bone which is eventually degraded and substituted by newly formed bone called creeping substitution. In the latter phase of this process, the degradation of the autologous bone liberates calcium phosphates and osteoinductive proteins, and enhances bone regeneration. The aim of the development of new bone substitutes is to provide an off the shelf solution to avoid an additional operation site for harvesting the autologous bone, pain and discomfort for the patient, donor site morbidity, and limitations in supply. During the first two phases, however, bone ingrowth from the defect margins heavily depend on the microarchitecture of the porosity, the material, and the surface of the bone substitute. The latter one was studied in depth for dental implants, since osseointegration of the implants is essential for their function. For titanium, the best surface appeared to be a moderately rough one. For calcium phosphate based bone substitutes submicron surface structures enhanced osteogenic differentiation compared to a micron scaled surface.

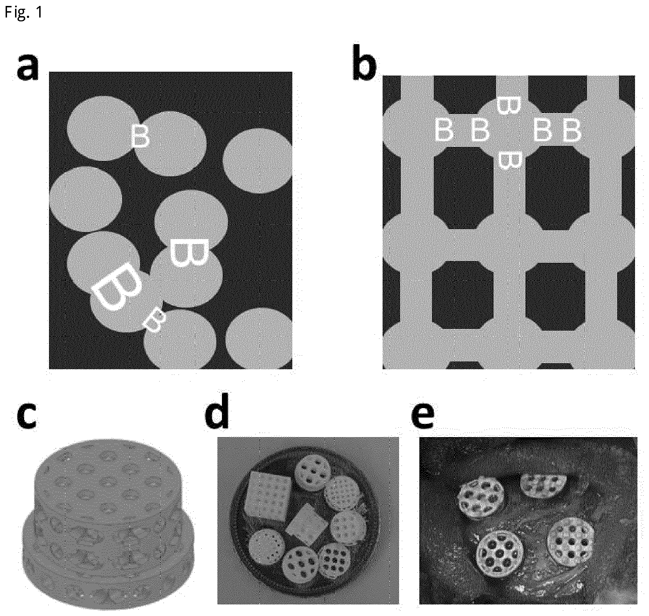

For decades, the microarchitecture as major driving force of osteoconduction determined as optimal pore size in bone substitute material was believed to be between 0.3-0.5 mm. These margins fall in line with the maximal optimal pore size for Haversian-type bone formation of 0.3-0.4 mm (Kuboki et al. (2001) The Journal of Bone & Joint Surgery 83, S105-S115) and the average pore size in trabecular bone of 0.2-0.4 mm (LeGeros et al. (2002) Clinical Orthopaedics and Related Research 395, 81-98). The majority of publications on optimal maximal pore size suggest the largest evaluated pore size to be optimal (Galois and Mainard (2004) Acta Orthop Belg 70, 598-603; 17; Murphy et al. (2010) Biomaterials 31, 461-466). In two publications, pores of 400-600 $\mu$m performed worse than the ones between 350-400 $\mu$m since pores in the range of 400-600 $\mu$m were occupied by adipocytes and bone marrow, causing the formation of multiple capillaries (Tsuruga et al. (1997) Journal of Biochemistry 121, 317-324) and translated to a reduction of the mechanical properties (Kuboki et. al., *ibid.*). The old dogma of the optimal pore size is based on observations of scaffolds with single channels or random distributed pores (Fig. 1a). Additive manufacturing opens up a new dimension of producing scaffolds with reproducible microarchitectures defining not only pore size, but also constrictions, like bottleneck dimensions (Fig. 1b, c, d).

The microarchitecture of bone substitutes generated by additive manufacturing / 3D printing is still chosen based on the old dogma of pore sizes between 0.3 and 0.5 mm (Carrel et al. (2016) Clin Oral Implants Res 27, 55-62; de Wild et al. (2013) Tissue Engineering Part A 19, 2645-2654) and efforts to unravel optimal pore dimensions for osteoconduction have not been undertaken with this new technology.

US2013/0195955A1 to Reichert and Hutmacher shows implant material based on polycaprolactone (PLC) filaments comprising bmp-7.

US2011/0307073 to Teoh et al. also shows a material based on layered grids of PCL filaments.

Based on the above-mentioned state of the art, the objective of the present invention is to provide means and methods to provide improved osteoconductive bone substitute materials. This objective is attained by the subject matter of the independent claims of the present specification.

The present invention was conceived as the result of an effort to design and produce scaffolds of defined pore sizes and bottleneck dimensions by lithography-based additive manufacturing with tricalcium phosphates and to test this library of scaffolds in vivo, to identify the most osteoconductive microarchitecture in terms of bone ingrowth into the scaffold determined by bony bridging of the defect and percentage of bony regenerated area.

Summary of the invention

The invention relates to an osteoconductive graft made of a biocompatible material forming a meshwork of trabeculae enclosing concave, spheroidal or spheric pores that are interspersed in the material as a network of non-overlapping pores connected by channels.

The pore-channel structure is generated by an additive manufacturing process building a graft body from two-dimensional layers at high geometric resolution (smaller than (<) 0,2 mm, particularly <0,1 mm). The graft material is composed of fine particles of a biocompatible material, held together for the manufacturing process by a polymerizable resin material (1% to 50%, particularly 5% to 35% w/w). The polymer is either removed from the graft material by heat treatment in a sintering step subsequent to the additive manufacturing process or is left in the graft if the polymer is biocompatible.

Detailed description of the invention

A first aspect of the invention relates to an osteoconductive graft comprising of a biocompatible material forming a meshwork of trabeculae enclosing pores that are interspersed in the material as a network of non-overlapping pores connected by channels. In certain embodiment, the osteoconductive graft is essentially made of the biocompatible material having the characteristic channel-pore structure described herein.

*Pore and channel geometry*

The pores are characterized by a pore diameter of 0,6 mm to 1,4 mm. In certain embodiments, the pores have a diameter of 0,7 mm to 1,3 mm. In certain particular embodiments, the pores have a diameter of 1,0 mm to 1,2 mm.

The channels are characterized by a channel diameter of 0.5 mm to 1.3 mm. In certain particular embodiments, the channels are characterized by a channel diameter of 0,7 mm to 1,2 mm. In any embodiment described herein, the channel diameter does not exceed the pore diameter. In certain particular embodiments, the channel diameter is smaller than the pore diameter by a factor of 0,95 or smaller. In certain particular embodiments, the channel diameter is at least 0,1mm, more particularly 0,2 mm or 0,3 mm smaller than the pore diameter.

A key feature of the invention is the spheroidal shape of the pores defining the material.

Each spheroidal pore is characterized by a diameter $d(x)$, $d(y)$ and $d(z)$ in each of the three dimensions of space. Absent production artefacts which may be introduced from time to time in a minority of pores, at least ($\geq$) 90% of pores (particularly for each pore, the relations of said diameters are

$d(x) = F\ d(y)$; $d(x) = G\ d(z)$; $d(y) = H\ d(z)$;

with F, G and H taking a value of 0,5 to 1,5. In other words, the diameter in the x axis is not less than half of the diameter in the y axis, but also not larger than one and a half time the diameter in the y axis, the same restriction applies for $d(x)$ with respect to the diameter in the z axis, and the diameter in the y axis and in the z axis also vary, at maximum, between 50% and 150%. It is understood that by defining two diameters, the boundary conditions for the third axis might be further restricted.

In certain particular embodiments, the axis diameters vary only between 80% and 125% of each other, in other words F, G and H taking a value of 0,8 to 1,25 with respect to each other. In certain more particular embodiments, the pores are characterized by an essentially spherical shape $[d(x) = d(y) = d(z)]$.

In the majority of bone substitutes, pores are distributed randomly. The present invention employs additive manufacturing, which facilitates defining the location of each single pore and to evaluate the perfect pore dimension. The spherical shape of pores enabled by the method of the invention enhances bone formation. Additionally, the mechanical properties of a microarchitecture based on spheroidal pores are superior to grid structures. Thirdly, the mechanical resistance to forces applies to all directions and shows a low anisotropy.

The pores are aligned in one, two or three spatial axes at regularly spaced intervals. The intervals (pore diameter plus channel separating pore from neighbouring pore) are defined by the pore diameter plus 0.3 mm to 1.3 mm. This microarchitecture was chosen to allow an undisturbed bone growth through the scaffold by aligning the pores in strings to achieve a high transparency of the scaffolds. The minimum distance between pores is ruled by the mechanics of the material and the restraints arising from the resolution of the additive manufacturing machine. In contrast to scaffolds produced by fiber deposition methodologies, which create convex surfaces, the microarchitecture of the present invention's material displays concave surfaces, known to enhance bone formation.

An alternative of this aspect of the invention relates to an osteoconductive graft comprising of, particularly essentially consisting of, a biocompatible material interspersed with a network of non-overlapping pores connected by channels, wherein the biocompatible material can be represented as a regular lattice of parallelepipeds as unit cells of the material. Certain particular embodiments of this aspect of the invention relate to rectangular parallelepipeds or cuboids as unit cell, the most particular unit cells are simple cubes.

Each of the unit cells contains a hollow spheroidal pore. The edges of the parallelepiped or cube are 0,8 mm to 2,7 mm in length. The distance of a face of the unit cell spheroid to the nearest point on a wall of the pore contained therein ranges between 0.15 and 0,65 mm. In certain embodiments, the distance is 0.3 to 1.0 mm.

The pores are connected by channels that can be characterized by their channel diameter, which ranges between 35 and 95% of the pore diameter in the projection along the axis of the channel.

In certain embodiments, the channel diameter is 0.5 mm to 1.3 mm. In certain particular embodiments, the channel diameter is 0.7 mm to 1.2 mm. The channel diameter does not exceed the pore diameter. The pores are aligned in one, two or three spatial axes at regularly spaced intervals, and the intervals (pore diameter plus channel separating pore from neighbouring pore) are defined by the pore diameter plus 0.3 mm to 1.3 mm.

In certain embodiments, the pores are regularly aligned to each other in one, two or three spatial axes. In certain particular embodiments, the pores are regularly aligned along two or three spatial axes that are perpendicular to each other.

In certain particular embodiments, ≥90% of the pores are characterized by a [for non-spheric spheroids: maximal] pore diameter that differs by ≤5% from the average of all pore diameters of said material. In embodiments wherein the spheroid is not an ideal sphere, this value relates to the average of the maximum diameter of the spheroids. In certain embodiments, the pore diameter differs by no more than 5% between said pores.

In certain embodiments, ≥90% of the channels are characterized by a channel diameter that differs by ≤5% from the average of all channel diameters of the material. In certain embodiments, the channel diameter differs by no more than 5% between said channels.

In certain embodiments, each of the pores in the bulk material, with the exception of the surface, is connected to six other pores by a channel. In certain embodiments, these six channels are oriented perpendicular to each other.

*Graft material*

In general, the graft is deposited by additive manufacturing methods. Any biocompatible inorganic material, bound by a polymerizable component, can be employed for the purpose of forming the graft. If the polymer must be removed from the graft prior to use, the body built by deposition of layers is a green body that is subsequently treated by heating it at a temperature, and for a time, sufficient to sinter the inorganic component and to remove the polymer. If the polymer is biocompatible, this sintering step is not required.

In certain embodiments, the osteoconductive graft material comprises or essentially consists of a bone substitute material selected from bio glass, hydroxyapatite, calcium phosphate, calcium sulfate, $CaCO_3$, particularly in the aragonite form, more particularly nacre, or a mixture thereof, provided in particulate form of sufficient size to allow additive manufacturing processes to process the material.

One advantage of the current invention is that it enables the forming of microporous structures characterized by concave pores for material that is not amenable to fibre extrusion, as practiced in the prior art. The carbonate or phosphate based materials mentioned above, for example, cannot be deposited as fibres.

Formation of the material of the invention by depositing entire contiguous layers, instead of deposition of fibre strands, brings the advantage that the layer thickness, and therefore the resolution of details such as pore or channel shape, can be significantly finer, and is only limited by the particle size of the material. The layers deposited in the examples given here ranges from 10 μm to 100 μm.

In certain embodiments, the osteoconductive graft material comprises or essentially consists of a bone substitute material selected from magnesium or a biocompatible magnesium alloy, particularly an Mg alloy having small (≤1%) additions of Zn, Fe, Ni and/or Cu, or a mixture thereof, provided in particulate form of sufficient size to allow additive manufacturing processes to process the material.

As bone predominantly is composed of calcium phosphate, a matrix manufactured from this material is amenable to the physiological processes of bone turnover that allow the body's own apparatus to dismantle and reconstruct the bone according to physiological needs.

Phosphate materials of particular utility for employment in the materials and methods disclosed herein include those of Table 1.

Table 1: Phosphate based mineral components

| Ca/P molar ratio | Compound | Formula |
| --- | --- | --- |
| 0,5 | Monocalcium phosphate monohydrate (MCPM) | $Ca(H_2PO_4)_2 * H_2O$ |
| 0,5 | Monocalcium phosphate anhydrous (MCPA) | $Ca(H_2PO_4)_2$ |
| 1,0 | Dicalcium phosphate dihydrate (DCPD), mineral brushite | $CaHPO_4 * 2\ H_2O$ |
| 1,0 | Dicalcium phosphate anhydrous (DCPA), mineral monetite | $CaHPO_4$ |
| 1,33 | Octacalcium phosphate (OCP) | $Ca_8(H_2PO_4)_2(PO_4)_4 * 5H_2O$ |

(continued)

| Ca/P molar ratio | Compound | Formula |
|---|---|---|
| 1,5 | α-Tricalcium phosphate (α-TCP) | $\alpha\text{-}Ca_3(PO_4)_2$ |
| 1,5 | β-Tricalcium phosphate (β-TCP) | $\beta\text{-}Ca_3(PO_4)_2$ |
| 1,0-2,2 | Amorphous calcium phosphate (ACP) | $Ca_xH_y(PO_4)_z * nH_2O$; n=3-4,5; 15-20% $H_2O$ |
| 1,5-1,67 | Calcium-deficient hydroxyapatite (CDHA) | $Ca_{10-x}(HPO_4)_x(PO_4)_{6-x}(OH)_{2-x}$; (0<x<1) |
| 1,67 | Hydroxyapatite (HA, HAp or OHAp) | $Ca_{10}(PO_4)_6(OH)_2$ |
| 1,67 | Fluorapatite (FA or FAp) | $Ca_{10}(PO_4)_6F_2$ |
| 1,67 | Oxyapatite (OA or OAp) | $Ca_{10}(PO_4)_6O$ |
| 2,0 | Tetracalcium phosphate (TTCP or TetCP), mineral hilgenstockite | $Ca_4(PO_4)_2O$ |

The inorganic material for making the graft may comprise, or essentially consist of, hydroxyapatite (HA). This material, however, has been found to be resorbed less efficiently by the body's own processes than other materials after sintering at above 1100°C, because of which for certain purposes, phosphate components have been developed that allow for better resorption after sintering. If HA is to be used and the graft body is to be sintered, the temperature of the sintering step is to be kept at temperatures below 1000°C in order to obtain graft material that can be resorbed by osteoclasts.

These include, without being limited to, so-called biphasic calcium phosphates (HA-β-tricalcium phosphate mixtures β-TCP, α-TCP, OCP, DCP and DCPD, see Table 1. Whereas HA, β-TCP, α-TCP and OCP are mostly resorbed by osteoclasts, at least DCPD but perhaps also DCP might be resorbed by simple dissolution.

CDHA and ACP may also be considered as inorganic component, as may any mixture of the materials shown in Table 1, alone or in combination with ceramic precursor materials, particularly silicon dioxide and/or sodium oxide.

One exemplary mixture of inorganic components is 40% HA and 60 % β-TCP.

In certain embodiments, the inorganic component of the graft material is a bio glass bone substitute material selected from the following compositions:

1) 46,1 $SiO_2$, 24,4 $Na_2O$, 26,9 CaO, 2,6 $P_2O_5$ (in mol-%)
2) 45S5 Hench bioglass: 45 wt.% $SiO_2$, 24.5 wt.% $Na_2O$, 24.5 wt.% CaO and 6 wt.% $P_2O_5$.
3) S53P4 Novabone also referred to as BoneAlive (clinically used) 53% $SiO_2$, 23% $Na_2O$ 20% CaO 4% $P_2O_5$
4) 13-93 53% $SiO_2$, 4% $P_2O_5$, 20% CaO, 5% MgO, 6% $Na_2O$ and 12% $K_2O$ (% in weight)

In certain embodiments, the bone substitute material is calcium phosphate. In certain particular embodiments, the bone substitute material is selected from tricalcium phosphate, hydroxyapatite and mixtures thereof.

The bone substitute material employed herein has certain advantages in comparison to titanium based implants, particularly with view to resorption, biocompatibility and the overall performance of the implant over longer durations. A significant part of the inventors' efforts was directed at overcoming the structural deficits of calcium phosphate and related materials, when compared to titanium. The latter allows realisation of trabeculae and other microstructures down to a size of 0,15 mm, which is not a size that can be realized for calcium salt structures due to their inferior mechanical qualities.

In certain embodiments, the osteoconductive graft material comprises the above bone substitute material and a tissue-compatible polymer embedding or binding the bone substitute material (particularly an acrylate-based polymer, more particularly polymethylmethacrylate). The polymer content is employed to allow shaping the fine structure of the graft by additive manufacturing methods. It is removed by high temperature treatment of the graft in many cases, but may be present before such treatment.

For reviews describing systems amenable to polymerization by light, see Decker, Macromol. Rapid Commun. 2002, 23, 1067-1093, or Steyrer et al., Materials 2017, 10, 1445; doi:10.3390/ma10121445. Systems for photopolymerization are well known in the art and generally include the monomer compounds generating the polymer, photoinitiators, which induce the curing reaction after exposure to light, and UV-stabilisators to prevent uncontrolled curing reactions.

In certain embodiments, the osteoconductive graft material comprises between 50 and 99 % (w/w) bone substitute material and between 1% and 50% (w/w) polymer. Certain embodiments comprise between 5% and 15% polymer.

Certain embodiments comprise between 10% and 25% polymer. Certain embodiments comprise between 15% and 35% polymer.

The calcination step to remove the polymer component is generally conducted at a temperature above 500°C, particularly from 700°C to 1200°C.

In certain embodiments, the bone substitute material is characterized by a grain size ranging from 0,1 $\mu$m to 100 $\mu$m. In certain embodiments, the particle size is chosen to range from 0,1 $\mu$m to 5 $\mu$m. In certain embodiments, the particle size is chosen to range from 3 $\mu$m to 25 $\mu$m. In certain embodiments, the particle size is chosen to range from 10 $\mu$m to 50 $\mu$m. In certain embodiments, the particle size is chosen to range from 30 $\mu$m to 100 $\mu$m. Careful selection of the particle size may allow fine-tuning of the resorption or dissolution kinetics of the material.

The osteoconductive graft material according to any one of the above claims, wherein the material is characterized by any one of the following geometrical parameters:

| Pore diameter [mm] | Channel diameter [mm] | Maximal Transparency [%] | Porosity [%] |
|---|---|---|---|
| 0.7 | 0.5 | 19.60 | 41.00 |
|  | 0.6 | 28.30 | 54.00 |
| 0.9 | 0.5 | 13.60 | 34.68 |
|  | 0.6 | 19.65 | 42.19 |
|  | 0.7 | 26.72 | 52.02 |
|  | 0.8 | 34.90 | 63.00 |
| 1.1 | 0.5 | 10.00 | 32.84 |
|  | 0.6 | 14.40 | 37.22 |
|  | 0.7 | 19.63 | 43.06 |
|  | 0.8 | 25.64 | 50.73 |
|  | 0.9 | 32.45 | 59.85 |
|  | 1.0 | 40.07 | 68.61 |
| 1.3 | 0.5 | 7.60 | 33.17 |
|  | 0.6 | 11.10 | 35.85 |
|  | 0.7 | 15.03 | 39.51 |
|  | 0.8 | 19.63 | 44.14 |
|  | 0,9 | 24.84 | 50.00 |
|  | 1.0 | 30.67 | 57.56 |
|  | 1.1 | 37.12 | 65.36 |
|  | 1.2 | 44.17 | 72.92 |

The values for porosity were calculated by the following formulae

$$\text{Porosity} = [1 - \rho_{\text{scaffold}} / \rho_{\text{materia}}] \times 100\%$$

wherein

$$\rho_{\text{scaffold}} = \frac{\text{mass}}{\text{volume}}$$

$\rho_{\text{scaffold}}$ is the density of the scaffold and $\rho_{\text{material}}$ is the density of the material of which the scaffold is fabricated. The volume is calculated by measuring the length, width, and height of different scaffolds.

The values for transparency were calculated by the formula as shown below

$$\text{Transparency} = \frac{\text{area of projection devoid of material}}{\text{total area of projection}}$$

The invention further relates to a method for making an osteoconductive graft comprising the steps of

a. Making a green body by depositing, in a 3-dimensional pattern, an inorganic particulate material selected from any one of the materials specified hereinabove, suspended in a photopolymerizable monomer, wherein said pattern is characterized by a network of non-overlapping spherical pores connected by channels, and wherein

i) said pores are characterized by a pore diameter of 0,6 mm to 1,4 mm, particularly 0,7 mm to 1,3 mm, more particularly 1,0 mm to 1,2 mm;
ii) said channels are characterized by a channel diameter of 0.5 mm to 1.3 mm, particularly 0,7 mm to 1,2 mm, wherein said channel diameter does not exceed said pore diameter [particularly wherein the channel diameter is smaller than the pore diameter by a factor of 0,95 or smaller], particularly wherein the channel diameter is at least 0,1mm [more particularly at least 0,2 mm or 0,3 mm] smaller than the pore diameter, and
iii) said pores are aligned in one, two or three spatial axes at regularly spaced intervals, wherein said intervals are defined by said pore diameter plus 0.3 mm to 1.3 mm
by subsequently depositing contiguous layers of said material, wherein each layer is between 10 $\mu$m and 100 $\mu$m;

b. and sintering said green body for 1-10 days at 500-1700°C.

In certain embodiments, the pores are characterized by a spheroidal shape, wherein each spheroidal pore is characterized by a diameter d(x), d(y) and d(z) in each of the three dimensions of space, and for $\geq$ 90% of pores (particularly for each pore), the relations of said diameters are

$$d(x) = F\ d(y);\ d(x) =\ G\ d(z);\ d(y) = H\ d(z);$$

with F, G and H taking a value of 0,5 to 1,5, particularly with F, G and H taking a value of 0,8 to 1,25, more particularly with said pores being characterized by an essentially spherical shape [d(x) = d(y) = d(z)].

Another aspect of the invention relates to a system comprising device for added manufacturing, in other words a 3-D printer, and a microprocessor controlling said device for added manufacturing. The printer is designed and equipped to deposit subsequent layers of a material as specified herein. It must be capable of handling essentially inorganic material, depositing this material at a resolution of 10-100 $\mu$m, particularly 20 to 50 $\mu$m, in each axis. The microprocessor is connected to the printer and runs it. It is programmed to deposit the material as a network of non-overlapping pores connected by channels as specified herein above.

Wherever alternatives for single separable features are laid out herein as "embodiments", it is to be understood that such alternatives may be combined freely to form discrete embodiments of the invention disclosed herein.

The invention is further illustrated by the following examples and figures, from which further embodiments and advantages can be drawn. These examples are meant to illustrate the invention but not to limit its scope.

Brief description of the figures

Fig. 1    shows a schematic drawing of bone substitutes, design, diverse scaffolds, and in vivo testing of tri-calcium phosphate based scaffolds. Porosogen based scaffolds pore distribution and bottleneck dimension (B) is shown (a) for porosogen based porous scaffolds. Scaffold in dark blue and pores in grey. Additively manufactured scaffolds (b) show far more uniform pore distribution and bottleneck dimension. (c) Design of one scaffold from the library. (d) Realization of diverse scaffolds. Scaffolds diverse in pore size and bottleneck dimension are displayed on a five-franc coin with 32 mm in diameter. (e) Screening model. Intra operative view on four scaffolds placed into 4 defects created in the calvarial bone of a rabbit.

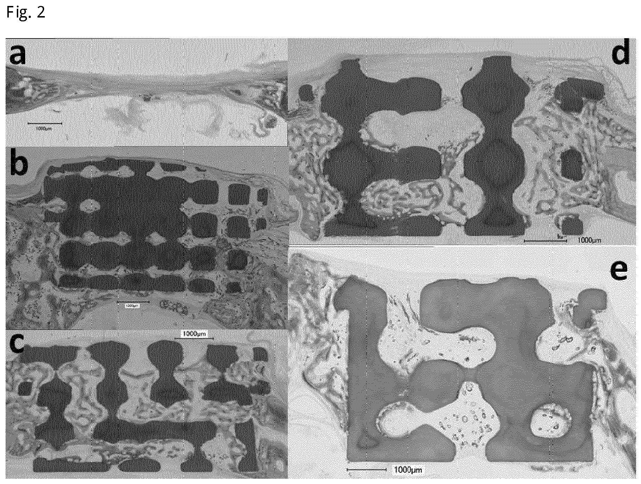

Fig. 2    shows toluidine-blue stained middle sections from untreated and scaffold treated defects:

(a) Empty control defect;

(b) Additively manufactured scaffold. Pore diameter of 0.5 mm and bottleneck diameter of 0.5 mm.
(c) Additively manufactured scaffold. Pore diameter of 0.7 mm and bottleneck diameter of 0.5 mm.
(d) Additively manufactured scaffold. Pore diameter of 1.2 mm and bottleneck diameter of 1.0 mm.
(e) Additively manufactured scaffold. Pore diameter of 1.5 mm and bottleneck diameter of 1.0 mm.

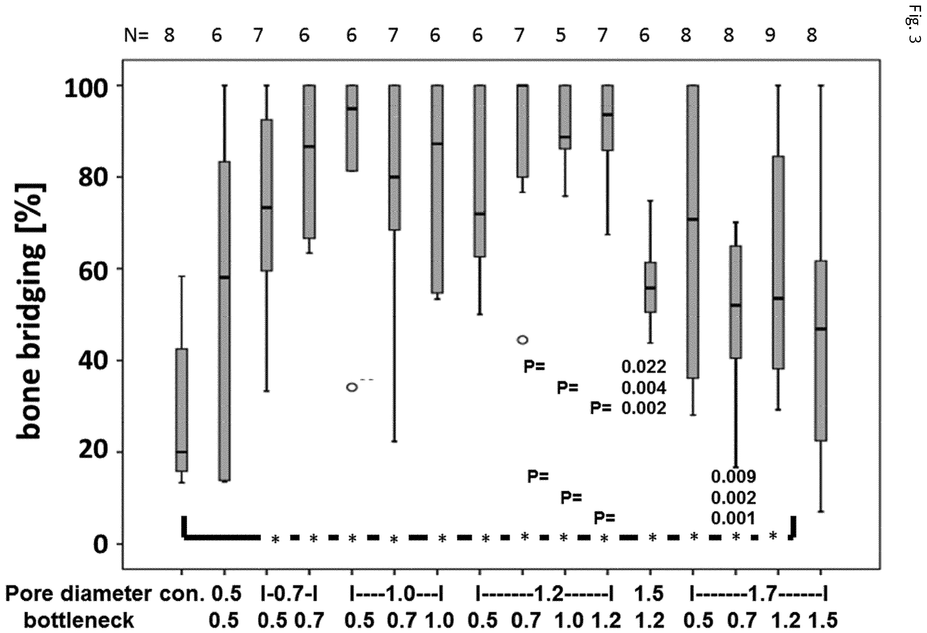

Fig. 3 shows bone bridging in relation to pore and bottleneck dimension. In comparison to the empty control, scaffolds with pore diameters from 0.7 to 1.7 mm and bottlenecks below 1.5 mm perform significantly better. Scaffolds with pores of 1.2 mm and bottlenecks between 0.7 and 1.2 perform significantly better than scaffolds with a pore diameter of 1.5 and bottleneck of 1.2 and pore diameter of 1.7 and bottleneck of 0.7.

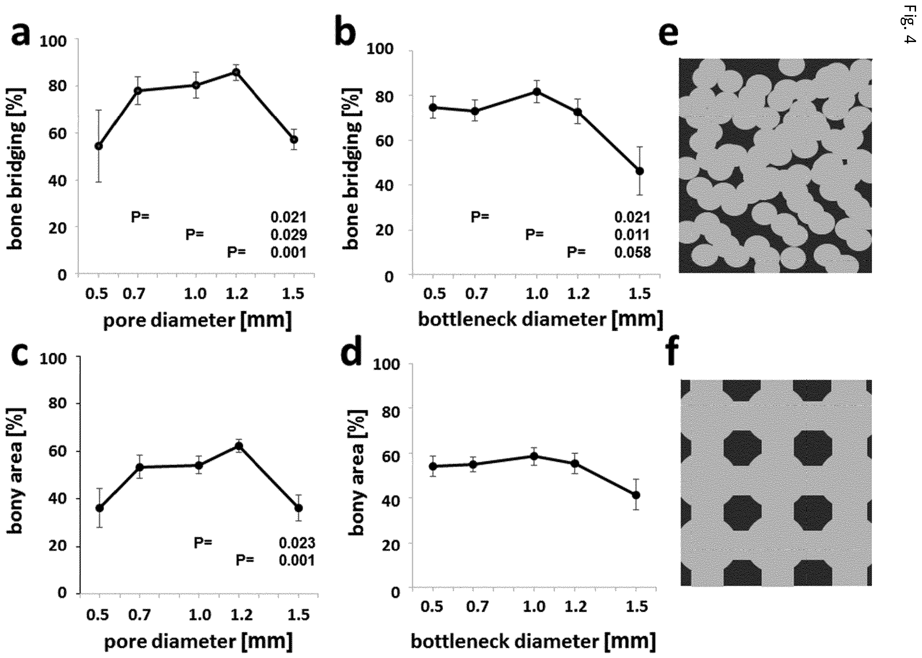

Fig. 4 shows the relationship of microarchitecture and osteoconduction. (a) Bony bridging and pore diameter. Bony bridging in relation to pore diameter of all groups. (b) Bony bridging and bottleneck diameter. Bony bridging in relation to bottleneck diameter of all groups. (c) Bony regenerated area and pore diameter. Bony regenerated area in relation to bottleneck diameter of all groups. (d) Bony regenerated area and bottleneck diameter. Bony regenerated area in relation to bottleneck diameter of all groups. (e) Schematic drawing of conventional scaffold based on random distributed 0.5 mm diameter pores. Scaffold in dark blue and pores in grey. (f) Schematic drawing of additively manufactured scaffold based on 1.2 mm pores and bottlenecks of 0.7 mm Scaffold in dark blue and pores in grey.

Examples

*Example 1: Scaffolds with pores between 0.7 and 1.2 mm perform better than empty controls*

The inventors employed the computer aided design software tool solidworks (Dassault Systèmes SolidWorks Corporation, Waltham, MA) to design a library of 20 different scaffolds to fit an established calvarial defect model system (de Wild et al., ibid.) where four non-critical size defects of 6 mm in diameter are created in calvarial bones of rabbits and used for screening purposes (Fig 1e). Five designs failed mechanically during production or during the *in vivo* testing and are not reported here. The different designs were fed to a lithography-based additive manufacturing machine (CeraFab7500, Lithoz, Vienna, Austria) to produce them in tri-calcium phosphate supplied as a Lithozbone™ (Lithoz, Vienna, Austria). After debindering of the scaffolds to eliminate the photoactive binder system and sintering to densify the scaffold to increase the mechanical stability (Fig. 1d) the scaffolds were implanted (Fig. 1e) for four weeks. Histologies (Fig. 2) show that bone formation occurred in and around the scaffolds and that the sintered tri-calcium phosphate based scaffolds are biocompatible. Evaluation of osteoconduction was based on the toluidine-blue stained middle ground sections by the determination of bony bridging and bony regenerated area. Compared to non-treated empty defects, almost all scaffolds induced better bony bridging or bony regeneration (Fig. 2). Only if the pore diameter was 1.5 mm and beyond, bony bridging and bony regeneration was reduced significantly.
Based on bony bridging as a measure of osteoconduction via the velocity of bone ingrowth into scaffolds or defects it appears that scaffolds with pore diameters between 0.7 and 1.2 mm perform equally well (Fig. 3). Scaffolds with pores of 1.2 mm and bottlenecks from 0.7 to 1.2 mm perform significantly better than scaffolds with pores of 1.5 mm and bottleneck of 1.2 mm. The same applied to scaffolds of pores from 1.7 mm and bottleneck of 0.7 mm. This suggests that pore diameters should be below 1.5 mm to reach a high velocity in defect bridging.

*The optimal pore diameter and bottleneck dimension for an osteoconductive scaffold is between 0.7 and 1.2 mm*

The inventors went on to evaluate bony bridging and bony regenerated area dependency grouped by pore diameter and bottleneck dimension, and found bony bridging to be significantly more complete in scaffolds with pores of 0.7 to 1.2 mm compared to a pore diameter of 1.5 mm (Fig. 4a) or 1.7 mm. For bony regenerated area, a pore diameter of 1.0 and 1.2 appeared significantly superior to a pore diameter of 1.5 mm (Fig. 4c). Bony bridging and bony regenerated area of scaffolds with 0.5 mm pores were always in the range of scaffolds with 1.5 mm diameter pores and below scaffolds with pores of 0.7, 1.0, and 1.2 mm in diameter. Therefore, optimal pore diameter for both measures of osteoconductivity lays between 1.0 and 1.2 mm. If the bottleneck between pores is 0.7 or 1.0 mm, bony bridging is significantly higher than for bottlenecks of 1.5 mm in diameter (Fig. 4b). For bony regenerated area, no significant difference between the bottleneck dimensions was observed.

Discussion

The inventors produced a library of tri-calcium phosphate based scaffolds and tested *in vivo* the influence of pore and bottleneck dimensions on osteoconduction determined by bony bridging of the defect and percentage of bony regenerated area. They chose an *in vivo* model system, since osteoconduction is a complex phenomenon, not well understood, originally described by histologies and due to the fact that *in vivo* and *in vitro* data are contradictory to each other (Zadpoor (2015) Biomater Sci 3, 231-245). The complexity of osteoconduction might arise from an interplay between, materials, surfaces, and microarchitectures, affecting individual osteogenic cells but also overall vascularization and directional bone tissue growth. Due to shortcomings of present *in vitro* systems, an *in vivo* approach was believed mandatory. The focus of the study on which the present specification is based, was on the microarchitecture of the scaffold.

The results presented herein suggest that the optimal pore size for an osteoconductive bone tissue engineering scaffolds is in the range of 1.0 to 1.2 mm and the bottleneck between pores between 0.7 and 1.0 mm. These values double or even quadruple the size of optimal pores for osteoconduction, and will have a deep impact on the microarchitecture of future, now truly osteoconductive scaffolds in terms of defect bridging, directional bone in-growth into scaffolds (Fig. 4 e, f). Pores and bottlenecks of 1.5 mm reduce defect bridging significantly and might compromise bone regeneration to a point where non-unions are induced. Clinically, the major complication in bone regeneration and the cause of numerous costly revision surgeries are non-unions. Therefore, the development of truly osteoconductive bone substitutes might reduce the number of failed bone regeneration procedures by non-unions significantly. Pores of 0.5 mm appear to perform worse than pores between 0.7 and 1.2 mm in diameter. Since pores of 0.5 mm in this overall scaffold design are right at the limit of our additive manufacturing machine, a final conclusion should not been drawn based on this system. Here, we clearly show that pores and bottleneck dimension below 1.5 mm and preferentially between 1.0 and 1.2 mm are optimal for osteoconduction and set new margins for osteoconductive scaffold microarchitectures.

For osteoconduction, the inventors found the optimal pore size in the range of 1.0 to 1.2 mm, and certainly below 1.5 mm. One can speculate that these pore and bottleneck dimensions reflect a balance of the positive interactions of directionally growing bone tissue during the course of bridging a defect with the surface of the scaffold, and the negative interactions due to bone growth restrictions by the scaffold. That the positive effects outweigh the negative one is evident by the fact that compared to empty untreated defects, all implants with pores between 0.7 and 1.7 mm and bottlenecks between 0.5 and 1.2 mm performed significantly better (Fig 3).

In certain embodiments, the scaffolds presented herein consist of tri-calcium phosphate, which is advantageously used for scaffolds in bone tissue engineering, because it degrades faster than hydroxyapatite, the natural component of bone, but remains biodegradable even after sintering at temperatures above 1100 °C. Biodegradation, however, was not an issue in the test system employed herein, since even tri-calcium phosphate degrades in months and not during a 4-week period, as was studied in the model system.

Additive manufacturing of free form scaffolds not restricted by the filament dimension of extrusion based additive manufacturing methodologies proved useful to study the microarchitecture of osteoconductive bone substitutes. Previously, porous bone substitutes were mainly produced with the help of porosogens resulting in an at random pore distribution, uncontrollable bottleneck dimensions, and a pore diameter between 0.3 and 0.5 mm (Fig. 4e). Based on our results the pore diameter of osteoconductive bone substitutes was increased to 1.0-1.2 mm and the bottleneck dimension to 0.7 - 1.0 mm (Fig. 4f) to optimize bone bridging and extent of bony regenerated area. Both will most likely diminish the formation of non-unions. Moreover, additive manufacturing is key in the realization of reproducible osteoconductive microarchitectures, where the microarchitecture can be adjusted according mechanical needs. Combined with the patient specific bone defect dimension, additive manufacturing will be central to realize a personalized treatment of any surgical procedure, where predictable bone regeneration with a low chance for the occurrence of non-unions is needed.

## Materials and Methods

### Design and fabrication of scaffolds

The inventors used the computer aided design software tool solidworks (Dassault Systèmes SolidWorks Corporation, Waltham, MA) to design a library of 20 different stepped scaffolds with a diameter of 6 mm in the lower 3 levels and 7.5 mm in the upper level as previously reported (24) and illustrated in figure 1c,d. The fabrication of tri-calcium phosphate based scaffolds was performed by a CeraFab 7500 (Lithoz, Vienna, Austria), with LithaBone TCP 200 ($Ca_3(PO_4)_2$) as photosensitive slurry, consisting of tri-calcium phosphate powder of particle size in the range of 5-30 $\mu$m, acrylate-based monomer, organic solvent (polypropyleneglycol), light absorber and photoinitiator at 43% of solid loading. The CeraFab 7500 (Lithoz, Vienna, Austria) was used to solidify the slurry in a layer by layer fashion resulting in a scaffold green part with a resolution, of 25 $\mu$m in layer thickness and 50 $\mu$m in the x/y-plane. After production, the green parts were removed from the building platform, cleaned from undetached slurry, and underwent a thermal treatment process to remove the solvent, to decompose the polymeric binder and to sinter the samples. The program for thermal treatment was provided by the manufacturer, and included a final sintering step of 3h at 1100°C.

*Animal experiments*

All animal procedures were approved by the Animal Ethics Committee of the local authorities (Canton Zurich, 108/2012 and 115/2015) and performed in accordance with the ethics criteria contained in the bylaws of the Institutional Animal Care and Use Committee. After the acclimatization period, bone regeneration was determined at the calvarial bone of 50 rabbits (female, 26 week old, New Zealand white rabbit) as described earlier (Karfeld-Sulzer et al. (2014) J Tissue Eng Regen Med). Sample size was determined by power analysis.

*Histomorphometry*

The evaluation of all implants was performed from the middle section using image analysis software (Image-Pro Plus®; Media Cybernetic, Silver Springs, MD). The area of interest (AOI) was defined by the 6 mm defect dimension and the height of the implant, corrected for differences in height between groups of different pore dimension. We determined the area of new bone in the AOI as percent of bone and bony integrated scaffold in the AOI (bony area, %). For the empty control value, the average corrected area occupied by all scaffolds was taken into account.

*Bone bridging*

The determination of bone bridging was performed as reported earlier (Kruse et al. (2011) Clin Oral Implants Res 22, 506-511; Schmidlin et al. (2013) Clin Oral Implants Res 24, 149-157). In brief, areas with bone tissue were projected onto the x-axis. Next, the stretches of the x-axis where bone formation had occurred at any level were summed up and related to the defect width of 6 mm. Bone bridging is given in percentage of the defect width (6mm) where bone formation had occurred.

*Statistical analysis*

The primary analysis unit was the animal. For all parameters tested, treatment modalities were compared with a Kruskal-Wallis test, followed by Mann-Whitney signed rank test for independent data (IBM SPSS v.23). Significance was set at $P \leq 0.05$. Values are reported as either mean $\pm$ standard error of the mean or displayed in box-plots ranging from the 25th (lower quartile) to the 75th (upper quartile) percentile including the median and whiskers showing the minimum and maximum values.

**Claims**

1. An osteoconductive graft comprising of, particularly essentially consisting of, a biocompatible material, wherein said material is interspersed with a network of non-overlapping spheroidal pores connected by channels, and wherein

   a) said pores are **characterized by** a pore diameter of 0.6 mm to 1.4 mm, particularly 0,7 mm to 1,3 mm, more particularly 1,0 mm to 1,2 mm;
   b) said channels are **characterized by** a channel diameter of 0.5 mm to 1.3 mm, particularly 0,7 mm to 1,2 mm, wherein said channel diameter does not exceed said pore diameter [particularly wherein the channel diameter is smaller than the pore diameter by a factor of 0,95 or smaller], particularly wherein the channel diameter is at least 0,1mm [more particularly at least 0,2 mm or 0,3 mm] smaller than the pore diameter, and
   c) said pores are aligned in one, two or three spatial axes at regularly spaced intervals, wherein said intervals are defined by said pore diameter plus 0.3 mm to 1.3 mm.

2. An osteoconductive graft comprising of, particularly essentially consisting of, a biocompatible material interspersed with a network of non-overlapping pores connected by channels, and wherein

   a) said material can be represented as a regular lattice of parallelepipeds (particularly of rectangular parallelepipeds or cuboids, more particularly of **cubes**) each containing a hollow spheroidal pore, wherein the edges of said parallelepiped (**cube**) are 0,8 mm to 2,7 mm in length, and the distance of a face of the cube to the nearest point on a wall of said pore ranges between 0.15 and 0,65 mm, particularly 0.3 to 1.0 mm, and
   b) said pores are connected by channels, wherein the channels are **characterized by** a channel diameter

      ◦ ranging between 35 and 95% of the pore diameter;
      ◦ of 0.5 mm to 1.3 mm, particularly 0.7 mm to 1.2 mm, wherein said channel diameter does not exceed said pore diameter

c) said pores are aligned in one, two or three spatial axes at regularly spaced intervals, wherein said intervals (pore diameter plus channel separating pore from neighbouring pore) are defined by said pore diameter plus 0.3 mm to 1.3 mm.

3. The osteoconductive graft material according to claim 1 or 2, wherein said pores are regularly aligned to each other in one, two or three spatial axes, particularly along two or three spatial axes that are perpendicular to each other.

4. The osteoconductive graft material according to any one of the preceding claims, wherein said pores are **characterized by** a spheroidal shape, wherein each spheroidal pore is **characterized by** a diameter d(x), d(y) and d(z) in each of the three dimensions of space, and for ≥ 90% of pores (particularly for each pore), the relations of said diameters are

$$d(x) = F\ d(y);\ d(x) =\ G\ d(z);\ d(y) = H\ d(z);$$

with F, G and H taking a value of 0,5 to 1,5, particularly with F, G and H taking a value of 0,8 to 1,25, more particularly with said pores being **characterized by** an essentially spherical shape [d(x) = d(y) = d(z)].

5. The osteoconductive graft material according to any one of the preceding claims, wherein ≥90% of said pores are **characterized by** a pore diameter that differs by ≤5% from the average of all pore diameters of said material.

6. The osteoconductive graft material according to any one of the preceding claims, wherein ≥90% of said channels are **characterized by** a channel diameter that differs by ≤5% from the average of all channel diameters of said material.

7. The osteoconductive graft material according to any one of the preceding claims, wherein each of said pores is connected to six other pores by said channels.

8. The osteoconductive graft material according to any one of the preceding claims, comprising or essentially consisting of

   a. a bone substitute material selected from

   i) a calcium phosphate or a mixture of calcium phosphates, particularly any one of the materials of Table 1, or a mixture thereof,
   ii) a calcium sulfate,
   iii) a calcium carbonate,
   iv) a mixture of any of the foregoing i) to iii), said mixture optionally mixed with $Na_2O$ and/or $SiO_2$, and
   v) magnesium or a magnesium alloy,

   b. and optionally, a polymer (particularly an acrylate-based polymer, more particularly polymethylmethacrylate).

9. The osteoconductive graft material according to any one of the above claims, wherein said bone substitute material is calcium phosphate, in particular tricalcium phosphate, hydroxyapatite and mixtures thereof.

10. The osteoconductive graft material according to any one of the above claims 8 or 9, wherein the osteoconductive graft material comprises between 50 and 100% (w/w) bone substitute material and between 50 and 0% (w/w) polymer.

11. The osteoconductive graft according to any one of the above claims 8 to 10, wherein the bone substitute material is **characterized by** a grain size ranging from 0,1 μm to 100 μm, particularly 0,1 μm to 5 μm or 3 μm to 25 μm or 10 μm to 50 μm or 30 μm to 100 μm.

12. The osteoconductive graft material according to any one of the above claims, wherein the material is **characterized by** any one of the following geometrical parameters:

| Pore diameter [mm] | Channel diameter [mm] | Transparency [%] | Porosity [%] |
|---|---|---|---|
| 0.7 | 0.5 | 19.60 | 41.00 |
|  | 0.6 | 28.30 | 54.00 |
| 0.9 | 0.5 | 13.60 | 34.68 |
|  | 0.6 | 19.65 | 42.19 |
|  | 0.7 | 26.72 | 52.02 |
|  | 0.8 | 34.90 | 63.00 |
| 1.1 | 0.5 | 10.00 | 32.84 |
|  | 0.6 | 14.40 | 37.22 |
|  | 0.7 | 19.63 | 43.06 |
|  | 0.8 | 25.64 | 50.73 |
|  | 0.9 | 32.45 | 59.85 |
|  | 1.0 | 40.07 | 68.61 |
| 1.3 | 0.5 | 7.60 | 33.17 |
|  | 0.6 | 11.10 | 35.85 |
|  | 0.7 | 15.03 | 39.51 |
|  | 0.8 | 19.63 | 44.14 |
|  | 0,9 | 24.84 | 50.00 |
|  | 1.0 | 30.67 | 57.56 |
|  | 1.1 | 37.12 | 65.36 |
|  | 1.2 | 44.17 | 72.92 |

**13.** A method for making an osteoconductive graft comprising the steps of

a. Making a green body by depositing, in a 3-dimensional pattern, an inorganic particulate material selected from any one of the materials specified in claim 8.a, suspended in a photopolymerizable monomer, wherein said pattern is **characterized by** a network of non-overlapping spherical pores connected by channels, and wherein

i) said pores are **characterized by** a pore diameter of 0,6 mm to 1,4 mm, particularly 0,7 mm to 1,3 mm, more particularly 1,0 mm to 1,2 mm;
ii) said channels are **characterized by** a channel diameter of 0.5 mm to 1.3 mm, particularly 0,7 mm to 1,2 mm, wherein said channel diameter does not exceed said pore diameter [particularly wherein the channel diameter is smaller than the pore diameter by a factor of 0,95 or smaller], particularly wherein the channel diameter is at least 0,1mm [more particularly at least 0,2 mm or 0,3 mm] smaller than the pore diameter, and
iii) said pores are aligned in one, two or three spatial axes at regularly spaced intervals, wherein said intervals are defined by said pore diameter plus 0.3 mm to 1.3 mm

by subsequently depositing contiguous layers of said material, wherein each layer is between 10 $\mu$m and 100 $\mu$m;
b. and sintering said green body for 1-10 days at 500-1700°C.

**14.** The method according to claim 13, wherein said pores are **characterized by** a spheroidal shape, wherein each spheroidal pore is **characterized by** a diameter d(x), d(y) and d(z) in each of the three dimensions of space, and for ≥ 90% of pores (particularly for each pore), the relations of said diameters are

$$d(x) = F\ d(y);\ d(x) =\ G\ d(z);\ d(y) = H\ d(z);$$

with F, G and H taking a value of 0,5 to 1,5, particularly with F, G and H taking a value of 0,8 to 1,25, more particularly with said pores being **characterized by** an essentially spherical shape [d(x) = d(y) = d(z)].

**15.** A system for performing a method according to any one of claims 13 to 14, said system comprising device for added manufacturing and a microprocessor controlling said device for added manufacturing, wherein

a. said device for added manufacturing is designed and equipped to deposit subsequent layers of a material as specified in any one of claims 1 to 12, and
b. said microprocessor being programmed to deposit said material as a network of non-overlapping pores connected by channels as specified in any one of claims 1 to 12.

**Patentansprüche**

**1.** Ein osteokonduktives Transplantat, umfassend, insbesondere im Wesentlichen bestehend aus einem biokompatiblen Material, wobei besagtes Material mit einem Netzwerk von nicht überlappenden sphäroidalen Poren durchsetzt ist, die durch Kanäle verbunden sind, und wobei

a) besagte Poren **gekennzeichnet sind durch** einen Porendurchmesser von 0,6 mm bis 1,4 mm, insbesondere 0,7 mm bis 1,3 mm, insbesondere 1,0 mm bis 1,2 mm;
b) besagte Kanäle **gekennzeichnet sind durch** einen Kanaldurchmesser von 0,5 mm bis 1,3 mm, insbesondere 0,7 mm bis 1,2 mm, wobei besagter Kanaldurchmesser besagten Porendurchmesser nicht übersteigt [insbesondere wobei der Kanaldurchmesser um einen Faktor von 0,95 oder weniger kleiner ist als der Porendurchmesser], insbesondere wobei der Kanaldurchmesser mindestens 0,1 mm [insbesondere mindestens 0,2 mm oder 0,3 mm] kleiner ist als der Porendurchmesser, und
c) besagte Poren in einer, zwei oder drei räumlichen Achsen in regelmäßig beabstandeten Intervallen angeordnet sind, wobei besagte Intervalle durch besagten Porendurchmesser plus 0,3 mm bis 1,3 mm definiert sind.

**2.** Ein osteokonduktives Transplantat, umfassend, insbesondere im Wesentlichen bestehend aus einem biokompatiblen Material, das mit einem Netzwerk von nicht überlappenden Poren, die durch Kanäle verbunden sind, durchsetzt ist, und wobei

a) besagtes Material als ein regelmäßiges Gitter von Parallelpipeden (insbesondere von rechteckigen Parallelepipeden oder Quadern, insbesondere von **Würfeln**) dargestellt werden kann, die jeweils eine hohle sphäroidale Pore enthalten, wobei
die Kanten besagten Parallelepipeds (**Würfels**) 0,8 mm bis 2,7 mm lang sind, und
der Abstand einer Fläche des Würfels zum nächstgelegenen Punkt auf einer Wand besagter Pore zwischen 0,15 und 0,65 mm, insbesondere 0,3 bis 1,0 mm, beträgt und
b) besagte Poren durch Kanäle verbunden sind, wobei die Kanäle durch einen Kanaldurchmesser gekennzeichnet sind,

o der zwischen 35 und 95 % des Porendurchmessers liegt;
◦ der 0,5 mm bis 1,3 mm, insbesondere 0,7 mm bis 1,2 mm beträgt, wobei besagter Kanaldurchmesser besagten Porendurchmesser nicht überschreitet

c) besagte Poren in einer, zwei oder drei räumlichen Achsen in regelmäßig beabstandeten Intervallen angeordnet sind, wobei besagte Intervalle (Porendurchmesser plus Kanal, der Pore von benachbarter Pore trennt) durch besagten Porendurchmesser plus 0,3 mm bis 1,3 mm definiert sind.

**3.** Das osteokonduktive Transplantat-Material gemäß Anspruch 1 oder 2, wobei besagte Poren regelmäßig in einer, zwei oder drei räumlichen Achsen zueinander angeordnet sind, insbesondere entlang zwei oder drei räumlichen Achsen, die senkrecht zueinander stehen.

**4.** Das osteokonduktive Transplantat-Material gemäß einem der vorhergehenden Ansprüche, wobei besagte Poren **gekennzeichnet sind durch** eine sphäroidale Form, wobei jede sphäroidale Pore **gekennzeichnet ist durch** einen Durchmesser d(x), d(y) und d(z) in jeder der drei Raumdimensionen, und für ≥ 90 % der Poren (insbesondere für jede Pore) die Verhältnisse von besagten Durchmessern

$$d(x) = F\ d(y);\ d(x) =\ G\ d(z);\ d(y) = H\ d(z)\ \text{sind};$$

mit F, G und H, die einen Wert von 0,5 bis 1,5 annehmen, insbesondere mit F, G und H, die einen Wert von 0,8 bis 1,25 annehmen,
wobei die Poren insbesondere durch eine im Wesentlichen sphärische Form [d(x) = d(y) = d(z)] gekennzeichnet sind.

5. Das osteokonduktive Transplantat-Material gemäß einem der vorhergehenden Ansprüche, wobei ≥90% besagter Poren durch einen Porendurchmesser gekennzeichnet sind, der sich um ≤5% vom Mittel aller Porendurchmesser von besagtem Material unterscheidet.

6. Das osteokonduktive Transplantat-Material nach einem der vorhergehenden Ansprüche, wobei ≥90 % besagter Kanäle durch einen Kanaldurchmesser gekennzeichnet sind, der sich um ≤5 % vom Mittel aller Kanaldurchmesser von besagtem Material unterscheidet.

7. Das osteokonduktive Transplantat-Material gemäß einem der vorhergehenden Ansprüche, wobei jede der besagten Poren durch besagte Kanäle mit sechs anderen Poren verbunden ist.

8. Das osteokonduktive Transplantat-Material gemäß einem der vorhergehenden Ansprüche, umfassend oder im Wesentlichen bestehend aus

    a. ein Knochenersatz-Material, ausgewählt aus

        i) ein Calciumphosphat oder ein Gemisch von Calciumphosphaten, insbesondere eines der Materialien der Tabelle 1, oder ein Gemisch davon,
        ii) ein Calciumsulfat,
        iii) ein Calciumcarbonat,
        iv) ein Gemisch aus einem der vorstehenden Punkte i) bis iii), wobei das Gemisch gegebenenfalls mit $Na_2O$ und/oder $SiO_2$ gemischt ist, und
        v) Magnesium oder eine Magnesiumlegierung,

    b. und gegebenenfalls ein Polymer (insbesondere ein Polymer auf Acrylatbasis, insbesondere Polymethylmethacrylat).

9. Das osteokonduktive Transplantat-Material gemäß einem der vorhergehenden Ansprüche, wobei besagtes Knochenersatz-Material Calciumphosphat, insbesondere Tricalciumphosphat, Hydroxyapatit und Mischungen davon ist.

10. Das osteokonduktive Transplantat-Material gemäß einem der obigen Ansprüche 8 oder 9, wobei das osteokonduktive Transplantrat-Material zwischen 50 und 100% (w/w) Knochenersatz-Material und zwischen 50 und 0% (w/w) Polymer umfasst.

11. Das osteokonduktive Transplantat gemäß einem der obigen Ansprüche 8 bis 10, wobei das Knochenersatz-Material **gekennzeichnet ist durch** eine Korngröße im Bereich von 0,1 μm bis 100 μm, insbesondere 0,1 μm bis 5 μm oder 3 μm bis 25 μm oder 10 μm bis 50 μm oder 30 μm bis 100 μm.

12. Das osteokonduktive Transplantat-Material gemäß einem der obigen Ansprüche, wobei das Material **gekennzeichnet ist durch** einen der folgenden geometrischen Parameter:

| Porendurchmesser [mm] | Kanaldurchmesser [mm] | Transparenz [%] | Porosität [%] |
|---|---|---|---|
| 0.7 | 0.5 | 19.60 | 41.00 |
| | 0.6 | 28.30 | 54.00 |

(fortgesetzt)

| Porendurchmesser [mm] | Kanaldurchmesser [mm] | Transparenz [%] | Porosität [%] |
|---|---|---|---|
| 0.9 | 0.5 | 13.60 | 34.68 |
| | 0.6 | 19.65 | 42.19 |
| | 0.7 | 26.72 | 52.02 |
| | 0.8 | 34.90 | 63.00 |
| 1.1 | 0.5 | 10.00 | 32.84 |
| | 0.6 | 14.40 | 37.22 |
| | 0.7 | 19.63 | 43.06 |
| | 0.8 | 25.64 | 50.73 |
| | 0.9 | 32.45 | 59.85 |
| | 1.0 | 40.07 | 68.61 |
| 1.3 | 0.5 | 7.60 | 33.17 |
| | 0.6 | 11.10 | 35.85 |
| | 0.7 | 15.03 | 39.51 |
| | 0.8 | 19.63 | 44.14 |
| | 0,9 | 24.84 | 50.00 |
| | 1.0 | 30.67 | 57.56 |
| | 1.1 | 37.12 | 65.36 |
| | 1.2 | 44.17 | 72.92 |

**13.** Ein Verfahren zur Herstellung eines osteokonduktiven Transplantats umfassend die Schritte

a. Herstellung eines Grünkörpers durch Ablagern, in einem 3-dimensionalen Muster, eines anorganischen partikulären Materials, ausgewählt aus einem der in Anspruch 8.a angegebenen Materialien, suspendiert in einem photopolymerisierbaren Monomer,
wobei besagtes Muster **gekennzeichnet ist durch** ein Netzwerk von nicht überlappenden sphärischen Poren, die durch Kanäle verbunden sind, und wobei

i) besagte Poren **gekennzeichnet sind durch** einen Porendurchmesser von 0,6 mm bis 1,4 mm, insbesondere 0,7 mm bis 1,3 mm, insbesondere 1,0 mm bis 1,2 mm;
ii) besagte Kanäle **gekennzeichnet sind durch** einen Kanaldurchmesser von 0,5 mm bis 1,3 mm, insbesondere 0,7 mm bis 1,2 mm, wobei besagter Kanaldurchmesser besagten Porendurchmesser nicht übersteigt [insbesondere wobei der Kanaldurchmesser um einen Faktor von 0,95 oder weniger kleiner als der Porendurchmesser ist], insbesondere wobei der Kanaldurchmesser mindestens 0,1 mm [insbesondere mindestens 0,2 mm oder 0,3 mm] kleiner als der Porendurchmesser ist, und
iii) besagte Poren in einer, zwei oder drei räumlichen Achsen in regelmäßig beabstandeten Intervallen angeordnet sind, wobei besagte Intervalle definiert sind durch besagten Porendurchmesser plus 0,3 mm bis 1,3 mm durch anschließendes Ablagern zusammenhängender Schichten besagten Materials, wobei jede Schicht zwischen 10 $\mu$m und 100 $\mu$m beträgt.;

b. und Sintern besagten Grünkörpers für 1-10 Tage bei 500-1700°C.

**14.** Das Verfahren gemäß Anspruch 13, wobei die Poren durch eine sphäroidale Form gekennzeichnet sind, wobei jede sphäroidale Pore **gekennzeichnet ist durch** einen Durchmesser d(x), d(y) und d(z) in jeder der drei Raumdimensionen, und für $\geq$ 90% der Poren (insbesondere für jede Pore) die Beziehungen von besagten Durchmessern

$$d(x) = F \ d(y); \ d(x) = G \ d(z); \ d(y) = H \ d(z) \ sind;$$

mit F, G und H, die einen Wert von 0,5 bis 1,5 annehmen, insbesondere mit F, G und H, die einen Wert von 0,8 bis 1,25 annehmen,

insbesondere, wobei die Poren durch eine im Wesentlichen sphärische Form [d(x) = d(y) = d(z)] gekennzeichnet sind.

**15.** Ein System zur Durchführung eines Verfahrens gemäß einem der Ansprüche 13 bis 14, besagtes System umfassend eine Vorrichtung zur additiven Fertigung und einen Mikroprozessor, der besagte Vorrichtung zur additiven Fertigung steuert, wobei

a. besagte Vorrichtung zur additiven Fertigung ausgestaltet und ausgerüstet ist, um aufeinanderfolgende Schichten eines Materials wie in einem der Ansprüche 1 bis 12 angegeben abzulagern, und

b. besagter Mikroprozessor so programmiert ist, besagtes Material als ein Netzwerk von nicht überlappenden Poren abzulagern, die durch Kanäle verbunden sind, wie in einem der Ansprüche 1 bis 12 angegeben.

## Revendications

**1.** Greffe ostéoconductrice comprenant, notamment essentiellement constituée de, un matériau biocompatible, dans laquelle ledit matériau est entrecoupé d'un réseau de pores sphéroïdaux non chevauchants connectés par des canaux, et dans laquelle

a) lesdits pores sont **caractérisés par** un diamètre de pore de 0,6 mm à 1,4 mm, notamment 0,7 mm à 1,3 mm, plus particulièrement 1,0 mm à 1,2 mm ;

b) lesdits canaux sont **caractérisés par** un diamètre de canal de 0,5 mm à 1,3 mm, notamment 0,7 mm à 1,2 mm, dans laquelle ledit diamètre de canal ne dépasse pas ledit diamètre de pore [notamment dans laquelle le diamètre de canal est inférieur au diamètre de pore par un facteur de 0,95 ou inférieur], notamment dans laquelle le diamètre de canal est inférieur d'au moins 0,1 mm [plus particulièrement d'au moins 0,2 mm ou 0,3 mm] au diamètre de pore, et

c) lesdits pores sont alignés dans un, deux ou trois axes spatiaux à des intervalles régulièrement espacés, dans laquelle lesdits intervalles sont définis par ledit diamètre de pore plus 0,3 mm à 1,3 mm.

**2.** Greffe ostéoconductrice comprenant, notamment essentiellement constituée de, un matériau biocompatible entrecoupé d'un réseau de pores non chevauchants connectés par des canaux, et dans laquelle

a) ledit matériau peut être représenté en tant que réseau régulier de parallélépipèdes (notamment de parallélépipèdes rectangles ou cuboïdes, plus particulièrement de cubes) contenant chacun un pore sphéroïdal creux, dans laquelle

les bords dudit parallélépipède (cube) sont de 0,8 mm à 2,7 mm en longueur, et

la distance d'une face du cube au point le plus proche sur une paroi dudit pore va de 0,15 à 0,65 mm, notamment 0,3 à 1,0 mm, et

b) lesdits pores sont connectés par des canaux, dans laquelle les canaux sont **caractérisés par** un diamètre de canal

◦ allant de 35 à 95 % du diamètre de pore ;

◦ de 0,5 mm à 1,3 mm, notamment 0,7 mm à 1,2 mm, dans laquelle ledit diamètre de canal ne dépasse pas ledit diamètre de pore ;

c) lesdits pores sont alignés dans un, deux ou trois axes spatiaux à des intervalles régulièrement espacés, dans laquelle lesdits intervalles (diamètre de pore plus canal séparant le pore du pore voisin) sont définis par ledit diamètre de pore plus 0,3 mm à 1,3 mm.

**3.** Matériau de greffe ostéoconductrice selon la revendication 1 ou 2, dans lequel lesdits pores sont alignés régulièrement l'un par rapport à l'autre dans un, deux ou trois axes spatiaux, notamment le long de deux ou trois axes spatiaux qui sont perpendiculaires l'un à l'autre.

**4.** Matériau de greffe ostéoconductrice selon l'une quelconque des revendications précédentes, dans lequel lesdits pores sont **caractérisés par** une forme sphéroïdale, dans lequel chaque pore sphéroïdal est **caractérisé par** un diamètre d(x), d(y) et d(z) dans chacune des trois dimensions de l'espace, et pour ≥ 90 % des pores (notamment pour chaque pore), les relations desdits diamètres sont

$$d(x) = F \, d(y) \; ; \; d(x) = G \, d(z) \; ; \; d(y) = H \, d(z) \; ;$$

avec F, G et H prenant une valeur de 0,5 à 1,5, notamment avec F, G et H prenant une valeur de 0,8 à 1,25, plus particulièrement avec lesdits pores qui sont **caractérisés par** une forme essentiellement sphérique [d(x) = d(y) = d(z)].

5. Matériau de greffe ostéoconductrice selon l'une quelconque des revendications précédentes, dans lequel ≥90 % desdits pores sont **caractérisés par** un diamètre de pore qui diffère de ≤5 % de la moyenne de l'ensemble des diamètres de pore dudit matériau.

6. Matériau de greffe ostéoconductrice selon l'une quelconque des revendications précédentes, dans lequel ≥90 % desdits canaux sont **caractérisés par** un diamètre de canal qui diffère de ≤5 % de la moyenne de l'ensemble des diamètres de canal dudit matériau.

7. Matériau de greffe ostéoconductrice selon l'une quelconque des revendications précédentes, dans lequel chacun desdits pores est connecté à six autres pores par lesdits canaux.

8. Matériau de greffe ostéoconductrice selon l'une quelconque des revendications précédentes, comprenant ou essentiellement constitué de

   a. un matériau de substitut osseux sélectionné parmi

   i) un phosphate de calcium ou un mélange de phosphates de calcium, notamment l'un quelconque des matériaux du Tableau 1, ou un mélange de ceux-ci,
   ii) un sulfate de calcium,
   iii) un carbonate de calcium,
   iv) un mélange de l'un quelconque des matériaux i) à iii) précédents, ledit mélange étant mélangé en option avec $Na_2O$ et/ou $SiO_2$, et
   v) du magnésium ou un alliage de magnésium,

   b. et en option, un polymère (notamment un polymère à base d'acrylate, plus particulièrement du polyméthacrylate de méthyle).

9. Matériau de greffe ostéoconductrice selon l'une quelconque des revendications précédentes, dans lequel ledit matériau de substitut osseux est le phosphate de calcium, notamment le phosphate tricalcique, l'hydroxyapatite et des mélanges de ceux-ci.

10. Matériau de greffe ostéoconductrice selon l'une quelconque des revendications précédentes 8 ou 9, dans lequel le matériau de greffe ostéoconductrice comprend entre 50 et 100 % (p/p) de matériau de substitut osseux et entre 50 et 0 % (p/p) de polymère.

11. Greffe ostéoconductrice selon l'une quelconque des revendications précédentes 8 à 10, dans laquelle le matériau de substitut osseux est **caractérisé par** une taille de grain allant de 0,1 $\mu$m à 100 $\mu$m, notamment 0,1 $\mu$m à 5 $\mu$m ou 3 $\mu$m à 25 $\mu$m ou 10 $\mu$m à 50 $\mu$m ou 30 $\mu$m à 100 $\mu$m.

12. Matériau de greffe ostéoconductrice selon l'une quelconque des revendications précédentes, dans lequel le matériau est **caractérisé par** l'un quelconque des paramètres géométriques suivants :

| Diamètre de pore [mm] | Diamètre de canal [mm] | Transparence [%] | Porosité [%] |
|---|---|---|---|
| 0,7 | 0,5 | 19,60 | 41,00 |
| | 0,6 | 28,30 | 54,00 |
| 0,9 | 0,5 | 13,60 | 34,68 |
| | 0,6 | 19,65 | 42,19 |
| | 0,7 | 26,72 | 52,02 |
| | 0,8 | 34,90 | 63,00 |
| 1,1 | 0,5 | 10,00 | 32,84 |
| | 0,6 | 14,40 | 37,22 |
| | 0,7 | 19,63 | 43,06 |
| | 0,8 | 25,64 | 50,73 |
| | 0,9 | 32,45 | 59,85 |
| | 1,0 | 40,07 | 68,61 |
| 1,3 | 0,5 | 7,60 | 33,17 |
| | 0,6 | 11,10 | 35,85 |
| | 0,7 | 15,03 | 39,51 |
| | 0,8 | 19,63 | 44,14 |
| | 0,9 | 24,84 | 50,00 |
| | 1,0 | 30,67 | 57,56 |
| | 1,1 | 37,12 | 65,36 |
| | 1,2 | 44,17 | 72,92 |

**13.** Procédé de fabrication d'une greffe ostéoconductrice comprenant les étapes consistant à

a. réaliser un corps vert en déposant, en un motif tridimensionnel, un matériau particulaire inorganique sélectionné parmi l'un quelconque des matériaux spécifiés dans la revendication 8.a, en suspension dans un monomère photopolymérisable,
dans lequel ledit motif est **caractérisé par** un réseau de pores sphériques non chevauchants connectés par des canaux, et dans lequel

i) lesdits pores sont **caractérisés par** un diamètre de pore de 0,6 mm à 1,4 mm, notamment 0,7 mm à 1,3 mm, plus particulièrement 1,0 mm à 1,2 mm ;
ii) lesdits canaux sont **caractérisés par** un diamètre de canal de 0,5 mm à 1,3 mm, notamment 0,7 mm à 1,2 mm, dans lequel ledit diamètre de canal ne dépasse pas ledit diamètre de pore [notamment dans lequel le diamètre de canal est inférieur au diamètre de pore par un facteur de 0,95 ou inférieur], notamment dans lequel le diamètre de canal est inférieur d'au moins 0,1 mm [plus particulièrement d'au moins 0,2 mm ou 0,3 mm] au diamètre de pore, et
iii) lesdits pores sont alignés dans un, deux ou trois axes spatiaux à des intervalles régulièrement espacés, dans lequel lesdits intervalles sont définis par ledit diamètre de pore plus 0,3 mm à 1,3 mm en déposant ensuite des couches contiguës dudit matériau, dans lequel chaque couche est comprise entre 10 $\mu$m et 100 $\mu$m ;

b. et fritter ledit corps vert pendant 1 à 10 jours à une température de 500 à 1700°C.

**14.** Procédé selon la revendication 13, dans lequel lesdits pores sont **caractérisés par** une forme sphéroïdale, dans lequel chaque pore sphéroïdal est **caractérisé par** un diamètre d(x), d(y) et d(z) dans chacune des trois dimensions de l'espace, et pour ≥ 90 % des pores (notamment pour chaque pore), les relations desdits diamètres sont

$$d(x) = F\, d(y)\ ;\ d(x) = G\, d(z)\ ;\ d(y) = H\, d(z)\ ;$$

avec F, G et H prenant une valeur de 0,5 à 1,5, notamment avec F, G et H prenant une valeur de 0,8 à 1,25, plus particulièrement avec lesdits pores qui sont **caractérisés par** une forme essentiellement sphérique [$d(x) = d(y) = d(z)$].

**15.** Système pour la réalisation d'un procédé selon l'une quelconque des revendications 13 à 14, ledit système comprenant un dispositif pour fabrication additive et un microprocesseur commandant ledit dispositif pour fabrication additive, dans lequel

a. ledit dispositif pour fabrication additive est conçu et équipé pour déposer des couches subséquentes d'un matériau tel que spécifié dans l'une quelconque des revendications 1 à 12, et
b. ledit microprocesseur étant programmé pour déposer ledit matériau en tant que réseau de pores non chevauchants connectés par des canaux tel que spécifié dans l'une quelconque des revendications 1 à 12.

Fig. 1

Fig. 2

Fig. 3

Fig. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20130195955 A1, Reichert and Hutmacher
- US 20110307073 A, Teoh

### Non-patent literature cited in the description

- **KUBOKI et al.** *The Journal of Bone & Joint Surgery,* 2001, vol. 83, S105-S115
- **LEGEROS et al.** *Clinical Orthopaedics and Related Research,* 2002, vol. 395, 81-98
- **GALOIS ; MAINARD.** *Acta Orthop Belg,* 2004, vol. 70, 598-603
- **MURPHY et al.** *Biomaterials,* 2010, vol. 31, 461-466
- **TSURUGA et al.** *Journal of Biochemistry,* 1997, vol. 121, 317-324
- **CARREL et al.** *Clin Oral Implants Res,* 2016, vol. 27, 55-62
- **DE WILD et al.** *Tissue Engineering Part A,* 2013, vol. 19, 2645-2654
- **DECKER.** *Macromol. Rapid Commun.,* 2002, vol. 23, 1067-1093
- **STEYRER et al.** *Materials,* 2017, vol. 10, 1445
- **ZADPOOR.** *Biomater Sci,* 2015, vol. 3, 231-245
- **KARFELD-SULZER et al.** *J Tissue Eng Regen Med,* 2014
- **KRUSE et al.** *Clin Oral Implants Res,* 2011, vol. 22, 506-511
- **SCHMIDLIN et al.** *Clin Oral Implants Res,* 2013, vol. 24, 149-157